(19) Europäisches Patentamt
European Patent Office
Office européen des brevets

(11) Numéro de publication: 0 288 647 B1

(12) FASCICULE DE BREVET EUROPEEN

(45) Date de publication de fascicule du brevet: 08.04.92 (51) Int. Cl.5: C07D 295/08, A61K 31/445

(21) Numéro de dépôt: 87401002.8

(22) Date de dépôt: 30.04.87

# (54) Dérivés du [(pyrrolidinyl-1)-2éthoxy]-5 p-cymène, le procédé de préparation desdits dérivés et les médicaments contenant lesdits dérivés.

(43) Date de publication de la demande:
02.11.88 Bulletin 88/44

(45) Mention de la délivrance du brevet:
08.04.92 Bulletin 92/15

(84) Etats contractants désignés:
AT BE CH DE ES GB GR IT LI LU NL SE

(56) Documents cités:
EP-A- 0 062 596
EP-A- 0 173 634
EP-A- 0 179 009
FR-A- 2 192 811
GB-A- 1 325 795

(73) Titulaire: INSTITUT DE RECHERCHES CHIMIOUES ET BIOLOGIOUES APPLIOUEES (I.R.C.E.B.A.) Société à responsabilité limitée dite:
62, Grande-Rue
F-78490 Vicq(FR)

(72) Inventeur: Danree, Bernard
59 bis Boulevard Devaux
F-78300 Opoissy(FR)
Inventeur: Houziaux, Patrick
Chemin des Jonchères
Bazemont F-78580 Maule(FR)
Inventeur: Lacolle, Jean-Yves
Résidence du Parc
F-78860 Saint-nom-la-Breteche(FR)

(74) Mandataire: Combe, André et al
CABINET BEAU DE LOMENIE 55, rue d'Amsterdam
F-75008 Paris(FR)

EP 0 288 647 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

## Description

La présente invention concerne des dérivés du [(pyrrolidinyl-1)-2-éthoxy]-5 p-cymène ; elle concerne également un procédé de préparation de ces dérivés et les médicaments qui contiennent lesdits dérivés.

Les dérivés du [(pyrrolidinyl-1)-2-éthoxy]-5 p-cymène sont les produits nouveaux de formule

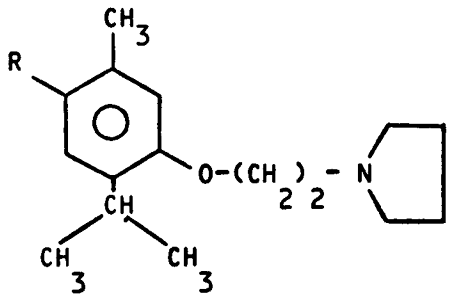

dans laquelle R est choisi parmi H, OH $OCOCH_3$ (acétoxy) et $OCO(CH_2)_nCH_3$, dans laquelle n est compris entre 1 et 8, (1 et 8 étant compris dans la définition de n) et les sels avec des acides pharmaceutiquement acceptables des produits de formule (I).

Le procédé de préparation des composés de formule (I) consiste dans un premier stade à faire réagir le thymol avec le chlorhydrate de la N-(chloro-2-éthyl)-pyrrolidine ; la réaction est réalisée par transfert de phase en système liquide-liquide en présence d'un catalyseur (chlorure de triéthylbenzylammonium) ; on obtient ainsi le produit de formule (I) dans lequel R est H. La préparation du produit de formule I dans lequel le groupe R est acétyle est réalisée dans un solvant tel que le toluène et en présence d'acide perchlorique à 70 % par réaction du produit dans lequel R = H avec de l'anhydride acétique.

La préparation du produit de formule (I) dans lequel le groupe R est acétoxy est réalisée par action sur le dérivé de formule (I)dans lequel R est acétyle, d'un oxydant tel que l'acide m-chloroperbenzoïque, ladite réaction étant effectuée dans un solvant (toluène) en présence d'un acide (acide trichloracétique).

La préparation du produit de formule (I) dans lequel le groupe R est un groupe hydroxy s'effectue par saponification, à l'aide d'une solution de soude, du produit dans lequel R est le groupe acétoxy.

Enfin, la préparation du produit de formule (I) dans laquelle le groupe R est $OCO(CH_2)_nCH_3$ s'effectue en réalisant l'estérification du produit dans lequel R est OH avec un chlorure d'acide de formule

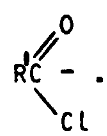

Les sels, par exemple les chlorhydrates, sont obtenus de façon connue en mettant en contact une solution du prduit de formule (I) avec l'acide considéré (par exemple en faisant barboter de l'acide chlorhydrique dans une solution du produit de formule (I).

La présente invention concerne également les médicaments caractérisés en ce qu'ils contiennent, comme produit actif, au moins un produit de formule (I) ; lesdits médicaments peuvent être utiles notamment dans le domaine de l'urologie.

Les exemples non limitatifs suivants illustrent les procédés de préparation des produits selon l'invention.

EXEMPLE 1
Synthèse du chlorhydrate du [(pyrrolidinyl-1)-2-éthoxy]-3 p-cymène (B 1007).

1. Préparation du [(pyrrolidinyl-1)-2-éthoxy]-3 p-cymène

Dans un tricol de 4 litres, muni d'un réfrigérant, d'une agitation pneumatique et d'un thermomètre, on introduit :
150,2 g (1 mole) de thymol
16,68 g de chlorure de triéthylbenzylammonium ; et

751 ml de lessive de soude.

On y ajoute 1 495 ml de chlorure de méthylène. Le milieu est agité très énergiquement.

L'addition de 212,76 g du chlorhydrate de la N-(chloro-2-éthyl)pyrrolidine (1,25 mole) dans 70,9 ml d'eau entraîne une élévation de température jusqu'à 25 °C.

Le mélange est chauffé au reflux pendant 4 heures sous vive agitation.

Après refroidissement à température ambiante, on laisse décanter la phase organique, on extrait la phase sodique par 2 × 425 ml de chlorure de méthylène.

Les phases organiques réunies sont lavées successivement par 2 × 350 ml d'eau acidulée (acide acétique 0,25 %), puis 2 × 700 ml d'eau saturée en chlorure de sodium jusqu'à neutralité. On les sèche ensuite sur sulfate de sodium.

Après filtration, le solvant est chassé sous vide.

On obtient 259,57 g d'une huile orangée.

Le produit brut est purifié par distillation fractionnée sous vide (sous azote).

On isole 184,7 g d'une huile incolore. Température d'ébullition sous 0,1 mmHg : 155-160 °C ; titre perchlorique/$CH_3COOH$ : 102,1 %.

2. Préparation du chlorhydrate

24,73 g de cette huile (0,1 mole) sont dissous dans 400 ml d'éther éthylique anhydre. La solution est saturée par un courant gazeux d'HCl sec sur un bain de glace. Les cristaux formés sont filtrès sur verre fritté, lavés par de l'éther éthylique anhydre, puis séchés sur potasse à 70 °C.

On obtient 25,12 g de cristaux beiges (rendement brut = 88,6 %).

Après recristallisation dans l'acétate d'éthyle, on isole 22,22 g de cristaux légèrement beiges.

Les cristaux obtenus ont été soumis l'analyse élémentaire (formule brute $C_{16}H_{26}ClNO$) qui a donné les résultats suivants :

| | calculé | trouvé |
|---|---|---|
| C | 67,70 | 67,69 |
| H | 9,23 | 9,25 |
| N | 4,93 | 4,83 |
| Cl | 12,48 | 12,65 |
| O | 5,63 | 5,76 |

Les cristaux ont un point de fusion : $F_{BK}$ = 157-158 °C et présentent des spectres IR et RMN conformes à la structure proposée.

EXEMPLE 2

Synthèse de l'acétyl-2[(pyrrolidinyl-1)-2-éthoxy]-5 p-cymène.

On utilise comme produit de départ le [(pyrrolidinyl-1)-2-éthoxy]-3 p-cymène obtenu selon le procédé 1 de l'exemple 1 ; ce produit a été au préalable purifié.

247,38 g (1 mole) de ce produit sont dissous dans 1 600 ml de toluène et 863 ml d'anhydride acétique dans un tricol de 4 litres muni d'un réfrigérant équipé d'une garde à $H_2SO_4$, d'un thermomètre et d'une ampoule à introduction. Le mélange est agité, puis on ajoute goutte à goutte, en maintenant la température inférieure à 45 °C, 225,2 ml d'acide perchlorique à 70 %.

Le mélange est agité durant 1 heure à température ambiante, puis versé sur 760 ml d'eau saturée en NaCl.

Après refroidissement par un bain de glace et basification par de la lessive de soude (pH : 12), on sépare la phase organique, on extrait par 2 x 300 ml de chlorure de méthylène. Les phases organiques sont réunies et lavées avec de l'eau acidulée et de l'eau saturée en NaCl jusqu'à neutralité. On sèche sur sulfate de sodium, on filtre et on chasse le solvant sous vide. On obtient 297,3 g d'une huile brune, d'une pureté C.P.V. de 97 % (rendement brut > 100 %).

Le produit brut est ensuite purifié par distillation fractionnée sous vide (sous azote).

On isole 196,14 g d'une huile jaune.

Le produit obtenu présente un point d'ébullition (sous 0,4 mmHg) de 149-153 °C, un titre perchlorique de 98,6 % et des spectres IR et RMN conformes à la structure proposée.

EXEMPLE 3
Synthèse du chlorhydrate de l'acétoxy-2[(pyrrolidinyl-1)-2-éthoxy]-5p-cymène. (B 1024).

1. Synthèse de l'acétoxy-2[(pyrrolidinyl-1)-2-éthoxy]-5 p-cymène

On utilise comme produit de départ l'huile purifiée obtenue dans l'exemple 2.

289,42 g (1 mole) de ce produit sont introduits avec 1 650 ml de toluène dans un tricol de 4 litres muni d'un réfrigérant équipé d'une garde à $H_2SO_4$, d'un thermomètre et d'une agitation pneumatique.

392,13 g (2,4 moles) d'acide trifluoracétique sont ajoutés par portions, en maintenant la température inférieure à 15 ° C. On introduit alors 258,84 g (1,2 mole) d'acide m-chloroperbenzoïque à 80 %.

Le mélange est maintenu 24 h à 15 ° C sous agitation. On le verse ensuite sur 2 130 ml d'ammoniaque à 5 %.

On décante la phase organique que l'on sépare. On extrait la phase aqueuse par 2 fois 890 ml de toluène. Les phases organiques sont rassemblées, lavées avec 890 ml d'eau acidulée, puis 1 180 ml d'eau saturée en NaCl jusqu'à neutralité. On sèche sur sulfate de sodium, on filtre et on chasse le solvant sous vide.

On obtient 266,3 g d'une huile brune d'une pureté C.P.V. de 97,2 % (rendement brut = 87,2 %).

2. Synthèse de chlorhydrate

30,54 g (0,1 mole) de cette huile brute sont dissous dans 210 ml d'éther éthylique anhydre. On y fait barboter un courant gazeux d'HCl sec, sur un bain de glace. Les cristaux formés sont filtrès sur verre fritté, lavés par de l'éther éthylique anhydre, puis séchés sur potasse à 50 ° C sous vide.

On isole 16,74 g de cristaux beiges (rendement brut = 49 %).

Après recristallisation dans un mélange AcOET/EtOH, 20/1, on obtient 11,31 g de cristaux beige clair.

Lesdits cristaux présentent un point de fusion $F_{BK}$ de 182-183 ° C, un titre perchlorique de 100,8 % et des spectres IR et RMN conformes à la structure proposée.

EXEMPLE 4
Synthèse du chlorhydrate de l'hydroxy-2[(pyrrolidinyl-1)-2-éthoxy]-5 p-cymène. (B 1058).

On utilise comme produit de départ l'huile purifiée obtenue après la première opération de l'exemple 3.

30,5 g (0,1 mole) de cette huile sont introduits avec 110 ml d'éthanol dans un erlenmeyer de 500 ml muni d'un réfrigérant et d'une agitation magnétique.

110 ml de soude 1 N (0,11 mole) sont ajoutés à cette solution et le mélange est agité 24 h à température ambiante. L'éthanol est chassé sous vide ; le résidu est repris par 150 ml d'eau, extrait par 3 fois 180 ml de chlorure de méthylène.

Les phases organiques réunies sont lavées par de l'eau saturée en chlorure de sodium jusqu'à neutralité. On sèche sur sulfate de sodium et on chasse le solvant sous vide. On obtient 25,28 g d'une huile orangée (rendement brut = 96 %).

Après cristallisation dans le pentane à chaud et recristallisation dans l'hexane, on isole 21,54 g de cristaux blancs présentant une température de fusion $F_{BK}$ de 86-87 ° C.

Le produit obtenu a été transformé en sel (chlorhydrate) en opérant comme suit :

13,16 g (0,05 mole) de base purifiée sont dissous dans 200 ml d'éther éthylique anhydre sous agitation. Après barbotage d'un courant gazeux d'HCl sec, on isole les cristaux formés par filtration sur fritté.

Après lavage par de l'éther éthylique, séchage sous vide à 50 ° C, on obtient 14,54 g de cristaux beiges (rendement brut = 97 %).

Après recristallisation dans un mélange AcOET/EtOH (2/1), on isole 11,1 g de cristaux blancs.

Lesdits cristaux présentent un point de fusion $F_{BK}$ de 147-148 ° C et des spectres IR et RMN conformes à la structure proposée ; l'analyse élémentaire de ces cristaux (formule brute $C_{16}H_{26}ClNO_2$) a donné les résultats suivants :

| | calculé | trouvé |
|---|---|---|
| C | 64,09 | 64,08 |
| H | 8,74 | 8,78 |
| N | 4,67 | 4,65 |
| Cl | 11,82 | 11,96 |
| O | 10,67 | 10,86 |

EXEMPLE 5

Préparation d'un ester et de son chlorhydrate

A - Synthèse du butyryloxy-2-[(pyrrolidinyl)-2-éthoxy]-5 p-cymène

R = $CH_3$ - $(CH_2)_2$ -

Dans un tricol de 500 ml, muni d'un réfrigérant, d'une agitation pneumatique et d'un thermomètre, on introduit sous agitation :

- 26,3 g (0,1 mole) d' hydroxy-2-[(pyrrolidinyl-1)-2-éthoxy]-5 p-cymène
- 200 ml de benzène
- 10,6 g de triéthylamine (0,105 mole)

A cette solution, on ajoute : 11,2 g (0,105 mole) de chlorure d'acide butyrique.

Le milieu réactionnel est chauffé à 50 °C durant 20 heures.

L'évolution de la réaction est contrôlée par des analyses du milieu en chromatographie en phase vapeur.

Après refroidissement à température ambiante, verser sur 350 ml d'eau.

Décanter la phase benzénique et contre-extraire la phase aqueuse par 3 x 250 ml de benzène.

Les phases benzéniques réunies sont lavées par l'eau jusqu'à neutralité et séchées sur sulfate de sodium.

Après filtration, le solvant est chassé sous vide.

On obtient 31,5 g d'une huile brune.

Rendement brut = 94,5 %
Pureté C.P.V = 99,5 %
Titre perchlorique = 94,3 %
C.C.M. = Monotache

B - Synthèse du chlorhydrate du butyryloxy-2[(pyrrolidinyl1)-2-éthoxy]-5 p-cymène (B 1132)

16,67 g (0,05 mole) de cette huile sont dissous dans 180 ml d'éther éthylique anhydre. La solution est saturée par un courant gazeux d'HCl sec (sur un bain de glace). Les cristaux formés sont filtrès, lavés par de l'éther éthylique anhydre, puis séchés sur anhydride phosphorique à 50 °C.

On obtient 12,18 g de cristaux beiges (rendement brut = 65,8 %).

Après recristallisation dans l'isopropanol, on isole 10,04 g de cristaux légèrement beiges.

Rendement après recristallisation = 54,3 %
$F_{BK}$ = 189 - 190 °C
Titre T.B.A.H. = 97,8 %
Titre $AgNO_3$ = 97 %
Pureté C.P.V. = 99,8 %
IR = Conforme à la structure proposée
RMN = Conforme à la structure proposée
Karl Fischer (dosage de l'eau) = 0,2 %
C.C.M. = Monotache

En opérant comme dans l'exemple 5, on a préparé les chlorhydrates mentionnés dans le tableau récapitulatif ci-après :

TABLEAU RECAPITULATIF

| N° code | n | Formule brute (PM) | Rendement % | $F_{BK}$ ° C (solvant recrist.) |
|---|---|---|---|---|
| B 1125 | 1 | $C_{19}H_{30}ClNO_3$ (355,91) | 51,2 | 177 - 178 |
| B 1132 | 2 | $C_{20}H_{32}ClNO_3$ (369,94) | 54,3 | 189 - 190 (I.P.A) |
| B 1131 | 4 | $C_{22}H_{36}ClNO_3$ (397,99) | 50 | 156 - 157 (I.P.A) |
| B 1134 | 8 | $C_{26}H_{44}ClNO_3$ (454,10) | 45,4 | 148 - 149 (I.P.A) |

Les produits selon l'invention ont été étudiés en ce qui concerne leur toxicité et leurs propriétés pharmacologiques.

1. Toxicité

Les doses létales 50 suivantes ont été obtenues après administration orale (P.O.) et intraveineuse (I.V.) des substances chez la souris.

Les résultats obtenus sont consignés dans le tableau I.

TABLEAU I

| Substances | DL 50 -mg/kg$^{-1}$ | |
|---|---|---|
| | P.O. | I.V. |
| B 1007 | 330 | 75 |
| B 1024 | 100 | 18 |
| B 1058 | 80 | 17 |
| B 1125 | ≃ 300 | ND * |
| B 1131 | ≃ 550 | ND * |
| B 1132 | ≃ 500 | ND * |
| B 1134 | ≃ 400 | 36 |
| Thymoxamine | 300 | 72,5 |

* ND : non déterminée

2. Propriétés pharmacologiques

2.1. - Activité α-adrénolytique in vitro

Elle a été étudiée sur le canal déférent du rat et sur l'urètre du lapin.

Principe de la mesure :

La noradrénaline provoque des contractions du canal déférent isolé du rat et de l'urètre isolé du lapin. La présence dans le bain de l'organe de substances α-bloquantes antagonise ces contractions : l'utilisation de concentrations croissantes de substances α-bloquantes permet de calculer :

- la $pA_2$ des composés sur le canal déférent du rat ; la $pA_2$ étant le logarithme changé de signe de la concentration molaire du produit en présence de laquelle il faut multiplier par deux la concentration de noradrénaline pour obtenir le même effet qu'en l'absence du produit ;
- la $pD'_2$ des composés sur l'urètre du lapin ; la $pd'_2$ étant le logarithme changé de signe de la concentration molaire du produit en présence de laquelle l'activité contracturante de la noradrénaline est divisée par deux.

Résultats : les résultats obtenus sont consignés dans le tableau II.

TABLEAU II

| PRODUITS | Action α-bloquante vis-à-vis de la noradrénaline | |
|---|---|---|
| | sur canal déférent isolé $pA_2$ | sur urètre isolé $pD'_2$ |
| B 1007 | 6,74 | 6,88 |
| B 1024 | 6,98 | 6,38 |
| B 1058 | 7,18 | 6,69 |
| B 1125 | 7,14 | 6,25 |
| B 1131 | 6,98 | 6,12 |
| B 1132 | 7,35 | 6,0 |
| B 1134 | 6,57 | 6,23 |
| Thymoxamine | 7,25 | 7,03 |

Ces résultats montrent une activité α-bloquante intéressante pour les produits testés.

2.2. - Activité adrénolytique in vivo

2.2.1. Chez le rat

Principe de la mesure

La noradrénaline injectée à fortes doses par voie intraveineuse provoque la mort de 100 % des animaux dans les 15 minutes qui suivent son injection. La mort survient par oedème pulmonaire dû à l'hypertension artérielle induite par la stimulation des récepteurs adrénergiques. L'administration préalable de substances α-adrénolytiques par voie orale permet de réduire la toxicité de la noradrénaline. Les produits sont administrés par voie orale à des temps variables entre 30 minutes et 6 heures avant l'injection intraveineuse (I.V.) de noradrénaline (0,4 $mg/kg^{-1}$).

Résultats :

Les résultats obtenus sont consignés dans le tableau III.

TABLEAU III

| PRODUITS | Doses p.o. $mg/kg^{-1}$ | % de protection contre la mort | Temps d'administration des produits avant I.V. |
|---|---|---|---|
| B 1007 | 50 | 60 | 30 min |
| B 1024 | 25 | 70 | 30 min |
| B 1058 | 50 | 90 | 30 min |
| B 1125 | 25 | 60 | 30 min |
| B 1131 | 50 | 60 | 30 min |
| B 1132 | 50 | 90 | 30 min |
| B 1134 | 100 | 100 | 4 h |
| Thymoxamine | 50 | 80 | 30 min |

Ces résultats montrent que les produits testés exercent une protection efficace vis-à-vis de la toxicité de la noradrénaline.

2.2.2. Chez le lapin anesthésié

L'activité α-bloquante "in vivo" aux niveaux urétral et vasculaire chez le lapin anesthésié a été recherchée par administration intraveineuse des produits B 1007, B 1024, B 1058, B 1125 et B 1134.

Principe de la mesure

L'injection intraveineuse de noradrénaline provoque chez le lapin une augmentation dose dépendante des pressions artérielle et urétrale. Les substances α-bloquantes injectées par voie intraveineuse antagonisent en fonction des doses ces augmentations de pression. On calcule la dose inhibitrice 50 % (D.I. 50) définie comme étant la dose de produit provoquant une diminution de 50 % des effets de la noradrénaline sur les pressions artérielle et urétrale.

Résultats

Les résultats obtenus sont consignés dans le tableau IV.

TABLEAU IV

| PRODUITS | D.I. 50 ($mg/kg^{-1}$) vis-à-vis de l'hypertension | |
|---|---|---|
| | artérielle | urétrale |
| B 1007 | 7,63 | 0,34 |
| B 1024 | 4,14 | 0,23 |
| B 1058 | 1,22 | 0,13 |
| B 1125 | 5,95 | 0,36 |
| B 1134 | 6,17 | 0,48 |
| Thymoxamine | 2,72 | 0,5 |

Ces résultats montrent que les produits testés antagonisent l'augmentation de pression urétrale à des doses beaucoup plus faibles que celles nécessaires pour antagoniser l'augmentation de pression artérielle.

2.2.3. Spécificité urétrale chez le chien anesthésié

L'effet des produits B 1007, B 1024, B 1125 et B 1134, injectés par voie intraveineuse sur l'hyperpression urétrale neurogène et sur la pression artérielle, a été recherché chez le chien anesthésié.

Principe de la mesure

La stimulation électrique du nerf hypogastrique provoque une augmentation de la pression urétrale par libération de noradrénaline à partir des fibres sympathiques du nerf.

Les substances α-bloquantes antagonisent en fonction des doses ces augmentations de pression urétrale et provoquent une hypotension artérielle par action bloquante au niveau des récepteurs vasculaires.

On calcule la dose qui inhibe de 50 % (DI. 50) les effets de la stimulation du nerf hypogastrique sur la pression urétrale et la dose qui provoque une hypotension artérielle de 20 % (DE. 20).

Résultats

Les résultats obtenus sont consignés dans le tableau V.

TABLEAU V

| PRODUITS | Hypotension artérielle DE. 20 - $mg/kg^{-1}$ | Hyperpression urétrale neurogène DI. 50 - $mg/kg^{-1}$ |
|---|---|---|
| B 1007 | 2 | 0,35 |
| B 1024 | 8 | 0,07 |
| B 1125 | 12 | 0,09 |
| B 1134 | 30 | 0,11 |
| Thymoxamine | 0,27 | 0,09 |

Ces résultats montrent que les produits testés antagonisent l'augmentation de pression urétrale induite par stimulation du nerf hypogastrique à des doses très inférieures à celles qui provoquent un effet vasculaire hypotenseur.

Les essais toxicologiques et pharmacologiques montrent que les produits selon l'invention sont utilisables par voie orale ou par voie injectable en tant que médicament dans les pathologies urétrales fonctionnelles relevant d'un mécanisme sympathique.

## Revendications

### Revendications pour les Etats contractants suivants : BE, CH, DE, GB, IT, LI, LU, NL, SE

**1.** Produits nouveaux de formule

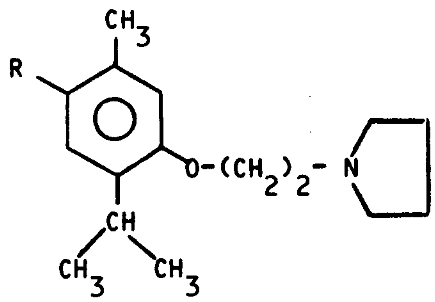

(I)

dans laquelle R est choisi parmi H, OH, $-OCOCH_3$ et $-O-CO-(CH_2)_n-CH_3$, dans laquelle n est compris entre 1 et 8 et les sels des produits de formule (I) avec des acides pharmaceutiquement acceptables.

**2.** Produits nouveaux selon la revendication 1, caractérisés en ce que n est choisi parmi 1, 4 et 8.

**3.** Procédé de préparation du produit de formule (I) dans lequel R est H, caractérisé en ce que l'on fait réagir le thymol avec le chlorhydrate de N-(chloro-2-éthyl)-pyrrolidine.

**4.** Procédé de préparation du produit de formule (I) dans lequel R est $-O-COCH_3$, caractérisé en ce que l'on fait réagir le produit de formule (I) dans lequel R est $-COCH_3$, lequel peut être obtenu en faisant réagir le produit de formule(I),dans lequel R est H avec de l'anhydride acétique en présence d'acide perchlorique, avec l'acide m-chloroperbenzoïque en présence d'un acide.

**5.** Procédé de préparation du produit de formule (I) dans lequel R est OH, caractérisé en ce que l'on fait réagir le produit de formula (I) dans lequel R est $-O-COCH_3$ avec une solution sodique.

**6.** Procédé de préparation des esters de formule (I), caractérisé en ce que l'on fait réagir l'hydroxy-2-[-(pyrrolidinyl-1)-2-éthoxy]-5 p-cymène sur un chlorure d'acide de formule

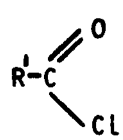

dans laquelle R' est $CH_3-(CH_2)_n$, n étant compris entre 1 et 8.

**7.** Médicaments présentant notamment une activité $\alpha$-bloquante, caractérisés en ce qu'ils contiennent au moins un produit de formule (I) ou un sel dudit produit.

### Revendications pour les Etats contractants suivants : AT, ES

**1.** Procédé de préparation d'un produit de formule :

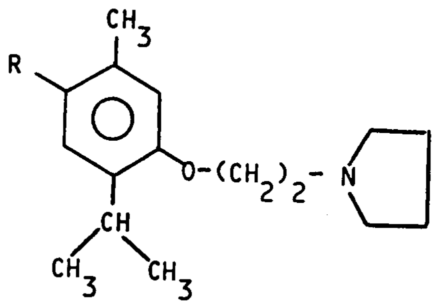

(I)

dans laquelle R est choisi parmi H, OH, $-OCOCH_3$ et $-O-CO-(CH_2)_n-CH_3$ dans laquelle n est compris entre 1 et 8 ou d'un sel d'addition de ces produits, caractérisé en ce qu'il comporte la préparation du produit de formule (I) dans laquelle R est H par réaction entre le thymol et le chlorhydrate de N(chloro-2-éthyl)-pyrrolidine, de préférence en présence d'un catalyseur de transfert de phase tel que le chlorure de triéthylbenzylammonium.

**2.** Procédé selon la revendication 1 pour la préparation du produit de formule (I) dans laquelle R est $-O-COCH_3$, caractérise en ce qu'il comprend la préparation précitée du produit de formule (I) dans laquelle R est H, la réaction de ce produit avec l'anhydride acétique, dans un solvant tel que le toluène et en présence d'acide perchlorique à 70 %, pour obtenir ainsi le produit de formule (I) dans lequel R est acétyle, et la réaction du produit ainsi obtenu avec l'acide m-chloroperbenzoïque en présence d'un acide.

**3.** Procédé selon la revendication 1 ou 2 pour la préparation du produit de formule (I) dans laquelle R est OH, caractérisé en ce qu'il comprend la réaction du produit de formule (I) dans laquelle R est $O-COCH_3$ avec une solution sodique.

**4.** Procédé de préparation selon l'une des revendications 1 à 3 des produits de formule (I) dans laquelle R est $-O-CO-(CH_2)n-CH_3$, caractérisé en ce qu'il comprend la réaction du produit de formule (I) dans laquelle R est OH avec un chlorure d'acide de formule :

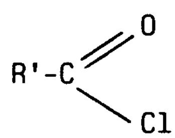

dans laquelle R' est $CH_3(CH_2)_n$, n étant compris entre 1 et 8

**Revendications pour l'Etat contractant suivant : GR**

**1.** Produits nouveaux de formule

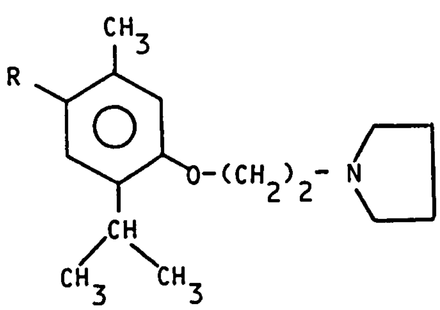

(I)

dans laquelle R est choisi parmi H, OH, $-OCOCH_3$ et $-O-CO-(CH_2)_n-CH_3$, dans laquelle n est compris entre 1 et 8 et les sels des produits de formule (I) avec des acides pharmaceutiquement acceptables.

**2.** Produits nouveaux selon la revendication 1, caractérisés en ce que n est choisi parmi 1, 4 et 8.

**3.** Procédé de préparation du produit de formule (I) dans lequel R est H, caractérisé en ce que l'on fait réagir le thymol avec le chlorhydrate de N-(chloro-2-éthyl)-pyrrolidine.

**4.** Procédé de préparation du produit de formule (I) dans lequel R est $-O-COCH_3$, caractérisé en ce que l'on fait réagir le produit de formule (I) dans lequel R est $-COCH_3$, lequel peut être obtenu en faisant réagir le produit de formule (I),dans lequel R est H avec de l'anhydride acétique en présence d'acide perchlorique, avec l'acide m-chloroperbenzoïque en présence d'un acide.

**5.** Procédé de préparation du produit de formule (I) dans lequel R est OH, caractérisé en ce que l'on fait réagir le produit de formule (I) dans lequel R est $-O-COCH_3$ avec une solution sodique.

**6.** Procédé de préparation des esters de formule (I), caractérisé en ce que l'on fait réagir l'hydroxy-2-[-(pyrrolidinyl-1)-2-éthoxy]-5 p-cymène sur un chlorure d'acide de formule

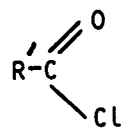

dans laquelle R' est $CH_3-(CH_2)_n$, n étant compris entre 1 et 8.

## Claims

**Claims for the following Contracting States : BE, CH, DE, GB, IT, LI, LU, NL, SE**

**1.** Novel products of the formula:

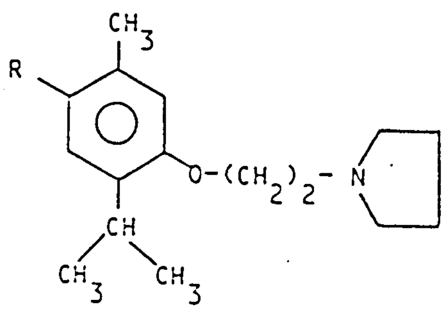

(I)

in which R is selected from the group comprising H, OH, $-OCOCH_3$ and $-O-CO-(CH_2)_n-CH_3$, in which n is between 1 and 8, and the salts of the products of the formula (I) with pharmaceutically acceptable acids.

**2.** Novel products according to claim 1, characterized in that n is selected from 1, 4 and 8.

**3.** Process for the preparation of the product of the formula (I) in which R is H, characterized in that thymol is reacted with N-(2-chloroethyl)pyrrolidine hydrochloride.

**4.** Process for the preparation of the product of the formula (I) in which R is $-O-COCH_3$, characterized in that the product of the formula (I) in which R is $-COCH_3$, obtainable by reacting the product of the formula (I) in which R is H with acetic anhydride in the presence of perchloric acid, is reacted with m-chloroperbenzoic acid in the presence of an acid.

**5.** Process for the preparation of the product of the formula (I) in which R is OH, characterized in that the product of the formula (I) in which R is $-O-COCH_3$ is reacted with a solution of sodium carbonate.

6. Process for the preparation of esters of the formula (I), characterized in that 2-hydroxy-5-[2-(pyrrolidin-1-yl)-ethoxy]-p-cymene is reacted with an acid chloride of the formula:

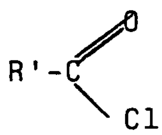

in which R' is $CH_3$-$(CH_2)_n$, n being between 1 and 8.

7. Drugs having especially an alpha-blocking activity, which contain at least one product of the formula (I) or a salt of the said product.

**Claims for the following Contracting States : AT, ES**

1. Process for the preparation of a product of formula:

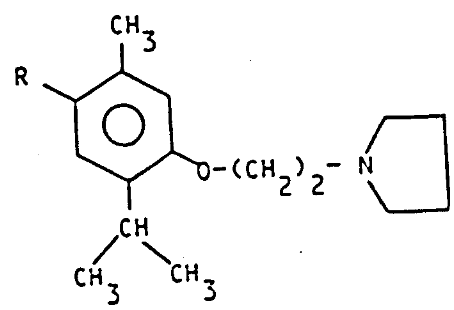

(I)

in which R is selected from the group comprising H, OH, $-OCOCH_3$ and $-O-CO-(CH_2)_n-CH_3$, in which n is between 1 and 8, or an addition salt of these products, characterized in that it comprises the preparation of the product of the formula (I), in which R is H, by reacting thymol with N-(2-chloroethyl)-pyrrolidine hydrochloride, preferably in the presence of a phase transfer catalyst, such as triethylbenzylammonium chloride.

2. Process according to claim 1 for the preparation of the product of formula (I) in which R is $-O-COCH_3$, characterized in that it comprises the precited preparation of the product of formula (I) in which R is H, the reaction of this product with acetic anhydide, in a solvent, such as toluene, and in the presence of 70% perchloric acid so as to obtain the product of formula (I) in which R is acetyl and the reaction of the thereby resulting product with the m-chloroperbenzoic acid in the presence of an acid.

3. Process according to claim 1 or 2 for the preparation of the product of formula (I) in which R is OH, characterized in that it comprises reacting the product of formula (I) in which R is $O-COCH_3$ with a sodium solution.

4. Process for the preparation according to one of claims 1 to 3 of the products of formula (I) in which R is $-O-CO-(CH_2)_n-CH_3$, characterized in that it comprises reacting the product of formula (I) in which R is OH with an acid chloride of the formula:

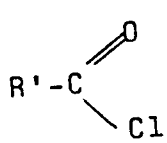

in which R' is $CH_3(CH_2)_n$, n being between 1 and 8.

**Claims for the following Contracting State : GR**

**1.** Novel products of the formula:

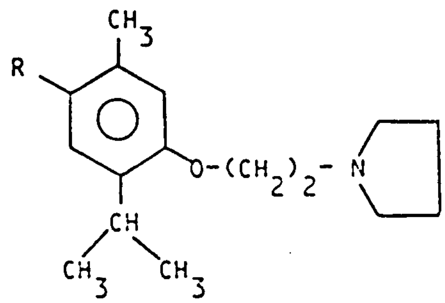

(I)

in which R is selected from the group comprising H, OH, $-OCOCH_3$ and $-O-CO-(CH_2)_n-CH_3$, in which n is between 1 and 8, and the salts of the products of the formula (I) with pharmaceutically acceptable acids.

**2.** Novel products according to claim 1, characterized in that n is selected from 1, 4 and 8.

**3.** Process for the preparation of the product of the formula (I) in which R is H, characterized in that thymol is reacted with N-(2-chloroethyl)pyrrolidine hydrochloride.

**4.** Process for the preparation of the product of the formula (I) in which R is $-O-COCH_3$, characterized in that the product of the formula (I) in which R is $-COCH_3$, obtainable by reacting the product of the formula (I) in which R is H with acetic anhydride in the presence of perchloric acid, is reacted with m-chloroperbenzoic acid in the presence of an acid.

**5.** Process for the preparation of the product of the formula (I) in which R is OH, characterized in that the product of the formula (I) in which R is $-O-COCH_3$ is reacted with a solution of sodium carbonate.

**6.** Process for the preparation of esters of the formula (I) , characterized in that 2-hydroxy-5-[2-(pyrrolidin-1-yl)-ethoxy]-p-cymene is reacted with an acid chloride of the formula:

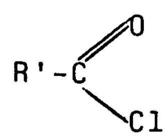

in which R' is $CH_3-(CH_2)_n$, n being between 1 and 8.

**Patentansprüche**

**Patentansprüche für folgende Vertragsstaaten : BE, CH, DE, GB, IT, LI, LU, NL, SE**

**1.** Neue Produkte der Formel

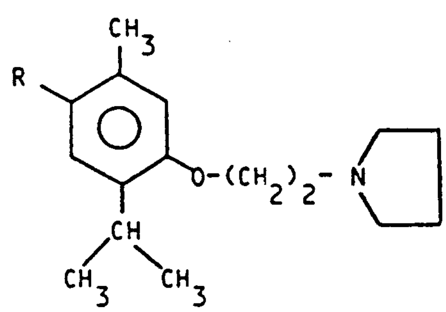

(I)

in welcher R ausgewählt ist aus H, OH, $-OCOCH_3$ und $-O-CO-(CH_2)_n-CH_3$, worin n zwischen 1 und 8 liegt, und die Salze der Produkte der Formel (I) mit pharmazeutisch akzeptablen Säuren.

**2.** Neue Produkte nach Anspruch 1, dadurch gekennzeichnet, daß n ausgewählt ist aus 1, 4 und 8.

**3.** Verfahren zur Herstellung des Produkts der Formel (I), in welcher R für H steht, dadurch gekennzeichnet, daß man Thymol mit N-(2-Chlorethyl)-pyrrolidin-hydrochlorid zur Umsetzung bringt.

**4.** Verfahren zur Herstellung des Produkts der Formel (I), in welcher R für $-O-COCH_3$ steht, dadurch gekennzeichnet, daß man das Produkt der Formel (I), in welcher R für $-COCH_3$ steht und welches durch Umsetzung des Produkts der Formel (I), in welcher R für H steht, mit Essigsäureanhydrid in Gegenwart von Perchlorsäure erhalten werden kann, mit m-Chlorbenzoesäure in Gegenwart einer Säure reagieren läßt.

**5.** Verfahren zur Herstellung des Produkts der Formel (I), in welcher R für OH steht, dadurch gekennzeichnet, daß man das Produkt der Formel (I), in welcher R für $-O-COCH_3$ steht, mit einer Sodalösung reagieren läßt.

**6.** Verfahren zur Herstellung der Ester der Formel (I), dadurch gekennzeichnet, daß man 2-Hydroxy-5-[2-(1-pyrrolidinyl)-ethoxy]-p-cymen mit einem Säurechlorid der Formel

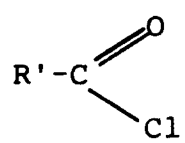 ,

in welcher R' für $CH_3-(CH_2)_n$ steht, wobei n zwischen 1 und 8 liegt, reagieren läßt.

**7.** Medikamente, die insbesondere eine $\alpha$-blockierende Aktivität aufweisen, dadurch gekennzeichnet, daß sie mindestens ein Produkt der Formel (I) oder ein Salz dieses Produkts enthalten.

**Patentansprüche für folgende Vertragsstaaten : AT, ES**

**1.** Verfahren zur Herstellung eines Produkts der Formel:

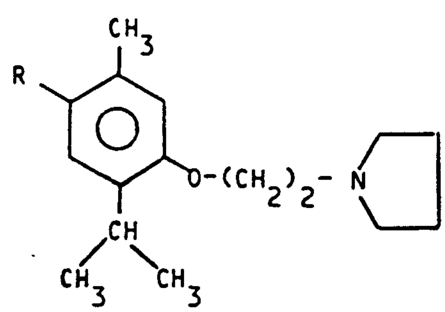 **(I)**

in welcher R ausgewählt ist aus H, OH, $-OCOCH_3$ und $-O-CO-(CH_2)_n-CH_3$, worin n zwischen 1 und 8 liegt, oder eines Additionssalzes dieser Produkte, dadurch gekennzeichnet, daß es die Herstellung des Produkts der Formel (I), in welcher R für H steht, durch Umsetzung von Thymol und N-(2-Chlorethyl)-pyrrolidin-hydrochlorid, vorzugsweise in Gegenwart eines Phasenumwandlungskatalysators, wie Triethylbenzylammoniumchlorid, umfaßt.

**2.** Verfahren nach Anspruch 1 zur Herstellung des Produkts der Formel (I), in welcher R für $-O-COCH_3$ steht, dadurch gekennzeichnet, daß es die Herstellung des Produkts der Formel (I), in welcher R für H steht, die Umsetzung dieses Produkts mit Essigsäureanhydrid in einem Lösungsmittel, wie Toluol, und in Gegenwart von 70 %iger Perchloressigsäure zur Darstellung des Produkts der Formel (I), bei welchem R Acetyl ist, und die Umsetzung des so erhaltenen Produkts mit m-Chlorperbenzoesäure in

Gegenwart einer Säure umfaßt.

**3.** Verfahren nach Anspruch 1 oder 2 zur Herstellung des Produkts der Formel (I), in welcher R für OH steht, dadurch gekennzeichnet, daß es die Umsetzung des Produkts der Formel (I), in welcher R für -O-$COCH_3$ steht, mit einer Sodalösung umfaßt. (I), in welcher R für O-$COCH_3$ steht, mit einer Sodalösung umfaßt.

**4.** Verfahren nach einem der Ansprüche 1 bis 3 zur Herstellung der Produkte der Formel (I), in welcher R für -O-CO-$(CH_2)_n$-$CH_3$ steht, dadurch gekennzeichnet, daß es die Umsetzung des Produkts der Formel (I), in welcher R für OH steht, mit einem Säurechlorid der Formel

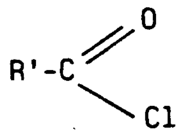

in welcher R' für $CH_3$-$(CH_2)_n$ steht, wobei n zwischen 1 und 8 liegt, umfaßt.

**Patentansprüche für folgenden Vertragsstaat : GR**

**1.** Neue Produkte der Formel

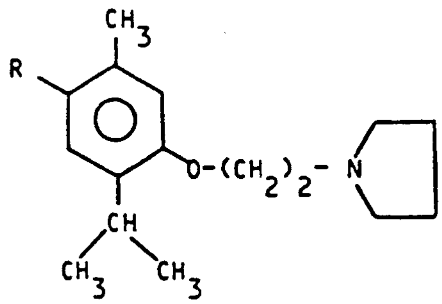

(I)

in welcher R ausgewählt ist aus H, OH, -$OCOCH_3$ und -O-CO-$(CH_2)_n$-$CH_3$, worin n zwischen 1 und 8 liegt, und die Salze der Produkte der Formel (I) mit pharmazeutisch akzeptablen Säuren.

**2.** Neue Produkte nach Anspruch 1, dadurch gekennzeichnet, daß n ausgewählt ist aus 1, 4 und 8.

**3.** Verfahren zur Herstellung des Produkts der Formel (I), in welcher R für H steht, dadurch gekennzeichnet, daß man Thymol mit N-(2-Chlorethyl)-pyrrolidin-hydrochlorid zur Umsetzung bringt.

**4.** Verfahren zur Herstellung des Produkts der Formel (I), in welcher R für -O-$COCH_3$ steht, dadurch gekennzeichnet, daß man das Produkt der Formel (I), in welcher R für -$COCH_3$ steht und welches durch Umsetzung des Produkts der Formel (I), in welcher R für H steht, mit Essigsäureanhydrid in Gegenwart von Perchlorsäure erhalten werden kann, mit m-Chlorbenzoesäure in Gegenwart einer Säure reagieren läßt.

**5.** Verfahren zur Herstellung des Produkts der Formel (I), in welcher R für OH steht, dadurch gekennzeichnet, daß man das Produkt der Formel (I), in welcher R für -O-$COCH_3$ steht, mit einer Sodalösung reagieren läßt.

**6.** Verfahren zur Herstellung der Ester der Formel (I), dadurch gekennzeichnet, daß man 2-Hydroxy-5-[2-(1-pyrrolidinyl)-ethoxy]-p-cymen mit einem Säurechlorid der Formel

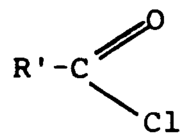,

in welcher R' für $CH_3$-$(CH_2)_n$ steht, wobei n zwischen 1 und 8 liegt, reagieren läßt.